# EUROPÄISCHE PATENTANMELDUNG

(11) **EP 2 679 239 A1**
(43) Veröffentlichungstag der Anmeldung: **01.01.2014**
(21) Anmeldenummer: 12173983.3
(22) Anmeldetag: 28.06.2012
(51) Int. Cl.: A61K 38/19, A61P 11/00

(54) **Pharmazeutische Zusammensetzung zur Behandlung der durch Sauerstoffarmut und verringerten Luftdruck vermittelten pulmonalen Form der Höhenkrankheit**

(71) Anmelder: Apeptico Forschung und Entwicklung GmbH, 1150 Wien (AT)
(72) Erfinder: Fischer, Bernhard., 1160 Wien (AT); Lucas, Rudolf., 30907 Martinez GA. (US); Fischer, Hendrik., 1160 Wien (AT)
(74) Vertreter: Sonn & Partner Patentanwälte

(57) **Zusammenfassung**

Beschrieben wird ein Peptid, welches aus 7-17 benachbarten Aminosäuren besteht und das Hexamer TX₁EX₂X₃E umfasst, wobei X₁, X₂ und X₃ jede natürliche oder nicht natürliche Aminosäure sein kann, wobei das Peptid keine TNF-Rezeptor-Bindungsaktivität aufweist und zyklisiert ist, zur Anwendung zur Behandlung und Vermeidung der pulmonalen Form der Höhenkrankheit.

SEQUENCE LISTING

<110>  APEPTICO Forschung und Entwicklung GmbH

<120>  Pharmazeutische Zusammensetzung zur Behandlung der durch
Sauerstoffarmut und verringerten Luftdruck vermittelten pulmonalen Form der
Höhenkrankheit

<130>  R62125

<160>  10

<170>  PatentIn version 3.5

<210>  1
<211>  17
<212>  PRT
<213>  Artificial Sequence

<220>
<223>  synthetic peptide

<400>  1

Cys Gly Gln Arg Glu Thr Pro Glu Gly Ala Glu Ala Lys Pro Trp Tyr
1               5                   10                  15

Cys

<210>  2
<211>  6
<212>  PRT
<213>  Artificial Sequence

<220>
<223>  synthetic peptide

<400>  2

Thr Pro Glu Gly Ala Glu
1               5

<210>  3
<211>  14
<212>  PRT
<213>  Artificial Sequence

<220>
<223>  synthetic peptide

<400>  3

Gln Arg Glu Thr Pro Glu Gly Ala Glu Ala Lys Pro Trp Tyr
1               5                   10

<210>  4
<211>  14
<212>  PRT
<213>  Artificial Sequence

<220>
<223>  synthetic peptide

<400>  4

Pro Lys Asp Thr Pro Glu Gly Ala Glu Leu Lys Pro Trp Tyr
1               5                   10


<210>  5
<211>  17
<212>  PRT
<213>  Artificial Sequence

<220>
<223>  synthetic peptide

<400>  5

Cys Gly Pro Lys Asp Thr Pro Glu Gly Ala Glu Leu Lys Pro Trp Tyr
1               5                   10                  15


Cys




<210>  6
<211>  17
<212>  PRT
<213>  Artificial Sequence

<220>
<223>  synthetic peptide

<400>  6

Cys Gly Gln Lys Glu Thr Pro Glu Gly Ala Glu Ala Lys Pro Trp Tyr
1               5                   10                  15


Cys




<210>  7
<211>  17
<212>  PRT
<213>  Artificial Sequence

<220>
<223>  synthetic peptide

<400>  7

Cys Gly Gln Arg Glu Thr Pro Glu Gly Ala Glu Ala Arg Pro Trp Tyr
1               5                   10                  15


Cys




<210>  8
<211>  15

<212>  PRT
<213>  Artificial Sequence

<220>
<223>  synthetic peptide

<400>  8

Cys Gly Gln Arg Glu Thr Pro Glu Gly Ala Glu Ala Lys Pro Cys
1               5                   10                  15


<210>  9
<211>  16
<212>  PRT
<213>  Artificial Sequence

<220>
<223>  synthetic peptide

<400>  9

Cys Gln Arg Glu Thr Pro Glu Gly Ala Glu Ala Lys Pro Trp Tyr Cys
1               5                   10                  15


<210>  10
<211>  17
<212>  PRT
<213>  Artificial Sequence

<220>
<223>  synthetic peptide

<400>  10

Cys Gly Gln Arg Glu Thr Pro Glu Gly Ala Glu Ala Lys Phe Trp Tyr
1               5                   10                  15


Cys

## Beschreibung

Die vorliegende Erfindung betrifft die Behandlung der durch Sauerstoffarmut und verringerten Luftdruck vermittelten pulmonalen Form der Höhenkrankheit.

Beim Menschen kann ab einer Höhe von oberhalb 2500 m über dem Meeresspiegel die Höhenkrankheit auftreten. In einer Höhe über 2500 Metern nehmen die Sauerstoffkonzentration und der Luftdruck deutlich ab. Man unterscheidet zerebrale und pulmonale Formen der akuten Höhenkrankheit. Die akute Höhenkrankheit ereignet sich also im Gehirn und auch in der Lunge.

Unbehandelt kann die pulmonale Form der Höhenkrankheit in weniger als 24 Stunden zum Tod führen, wobei der Tod häufig durch eine sekundäre Lungenembolie eintritt.

Die wirksamste Behandlung aller Formen der akuten Höhenkrankheit ist die Zufuhr von Sauerstoff, zum Beispiele durch schnellen Abstieg des Erkrankten in tiefere Höhenlagen, oder mittels Flaschensauerstoff oder mittels Überdrucksack. Jedoch ist in bergigen Gebieten ein schneller Abstieg oft nicht möglich. Sauerstoffbeatmung durch z.B. Flaschensauerstoff senkt zwar den erhöhten pulmonalarteriellen Druck, normalisiert ihn aber nicht. Auch beim Überdrucksack ist der positive Effekt nur zeitlich begrenzt. Der Therapieerfolg nach Verlassen des Überdrucksackes verschwindet sofort bei Patienten, wenn dieser wieder körperlich aktiv wird.

Eine medikamentöse Behandlung der Höhenkrankheit ist derzeit nur beschränkt und kontroversiell: So wird bei einer schweren Akuten Höhenkrankheit und auch speziell bei der zerebralen Form der Höhenkrankheit Dexamethason vorgeschlagen. Weiters wurde diskutiert, ob PDE-5-Hemmer, die zur Behandlung der primären pulmonal arteriellen Hypertonie eingesetzt werden (Dana Point-Klassifikation 1) auch für die sekundäre pulmonale Hypertonie durch Sauerstoffmangel in Höhen (Dana Point Klassifikation 3) indiziert sind.

Auch natürliche Behandlungsmöglichkeiten für Höhenkrankheit (auch vorbeugend) wurden vorgeschlagen (Tee aus Blättern des Cocastrauchs; Yak-Buttertee; Präparate, die Ginkgo als Wirkstoff enthalten).

Es ist aber festzustellen, dass derzeit die medikamentöse Behandlungsmöglichkeit der pulmonalen Form der Höhenkrankheit noch sehr eingeschränkt ist. Darüber hinaus ist bekannt, dass nur im europäischen alpinen und teilweise auch im nordamerikanischen Bereich mit organisierten Rettungseinsätzen gerechnet werden kann. In abgelegenen Hochgebirgen der Erde und in den extremen Höhen sind Rettungseinsätze und medizinische Hilfe bei Notfällen (mit Einsatz von Flaschensauerstoff oder Überdrucksack) kaum mehr möglich. Die Bereitstellung einer effizienten medikamentösen Behandlung der Höhenkrankheit wäre daher dringend erforderlich, auch als Bestandteil als Notfallpaket für Bergsteiger, die in Gefahr geraten können, die Höhenkrankheit zu entwickeln.

Aufgabe der vorliegenden Erfindung ist daher, die Möglichkeiten der medikamentösen Behandlung von Patienten mit der pulmonalen Form der Höhenkrankheit deutlich zu verbessern und ein Mittel zur Verfügung zu stellen, mit welchem diese Erkrankung effektiv behandelt, aber auch vermieden werden kann.

Demgemäß betrifft die vorliegende Erfindung ein Peptid, welches aus 7-17 benachbarten Aminosäuren besteht und das Hexamer TX₁EX₂X₃E umfasst, wobei X₁, X₂ und X₃ jede natürliche oder nicht natürliche Aminosäure sein kann, wobei das Peptid keine TNF-Rezeptor-Bindungsaktivität aufweist und zyklisiert ist, zur Behandlung und Vermeidung der pulmonalen Form der Höhenkrankheit.

Die erfindungsgemäß zu verwendenden Peptide sind an sich schon länger bekannt, beispielsweise aus dem europäischen Patent EP 1 264 599 B1, der US 2007/299003 A, WO 94/18325 A1 oder WO 2008/148545 A1. Im Zuge der Experimente zur vorliegenden Erfindung wurde nunmehr erkannt, dass sich diese Peptide überraschenderweise auch dazu eignen, die pulmonale Form der Höhenerkrankung zu behandeln, so dass somit erstmals eine einfache und effiziente medikamentöse Behandlungsform für diese Indikation zur Verfügung gestellt werden kann.

Diese - an sich bekannten - Peptide, die erfindungsgemäß zum Einsatz kommen, weisen keine TNF-Rezeptor-Bindungsaktivität auf (Hribar et al., Eur. J. Immunol. 1999; Elia et al., AJRCCM 2003; s. auch: Beispielteil unten) und sind zyklisiert. Bevorzugte Varianten dieser Peptide bestehen aus 7-17 benachbarten Aminosäuren und enthalten das Hexamer TPEGAE (SEQ ID Nr. 2).

Die akute Höhenkrankheit beginnt immer mit subakuter Hypoxie. In der Folge führen Hypoxämie und Hyperkapnie zu Vasodilatation, Hypokapnie zu Vasokonstriktion. In der Höhe ergeben sich nun aus Hypoxämie und Hypokapnie unterschiedliche Effekte: In der Lunge überwiegt Vasokonstriktion, im Gehirn Vasodilatation.

Die Ursache der akuten Höhenkrankheit liegt in einer fehlgeschlagenen Adaptation, vor allem in einer individuell zu geringen Ventilationssteigerung (relative Hypoventilation). Die Folgen sind ausgeprägtere Hypoxämie, höherer Pulmonalarteriendruck, höherer intrakranieller Druck, Flüssigkeitsretention und geringere Erythropoese.

Die pulmonale Form der Höhenkrankheit wird durch Sauerstoffarmut und verringerten Luftdruck vermittelt und ist eine lebensbedrohende Veränderung der Lungenfunktion und kommt vornehmlich in Höhen zwischen 2500 und 6000 m vor. Zwei Drittel aller Fälle ereignen sich zwischen 3000 und 4500 m Seehöhe. Die pulmonale Form der Höhenkrankheit ist die häufigste Todesursache der akuten Höhenkrankheit.

Die pulmonale Form der Höhenkrankheit beginnt oft charakteristischerweise nach Überschreiten der Schwellenhöhe von etwa 2500 m.

Die überschießende, inhomogene, hypoxische Vasokonstriktion in der Lunge führt zu überperfundierten Lungenbezirken mit akuten Infiltraten. Die massiv erhöhte pulmonale Hypertension als Folge einer inhomogenen hypoxischen Vasokonstriktion ist vor allem in peripheren Lungenarealen Ausdruck einer stark erhöhten hypoxisch-pulmonal-vaskularen Reaktion (HPVR, Hypoxic Pulmonary Vascular Response) bei vorher völlig gesunden Menschen. Eine Erhöhung des Pulmonalarteriendruckes ist unter Hypoxie zwar physiologisch, bei der pulmonalen Form der Höhenkrankheit aber deutlich höher ausgeprägt. Allerdings ist die pulmonale Kapillarpermeabilität unter Hypoxie nicht erhöht.

Dies steht im deutlichen Gegensatz zu anderen akuten Lungenerkrankungen, wie zum Beispiele dem Akuten Lungenschaden (ALI), dem Akuten Respiratorischen Distress Syndrom (ARDS) oder dem Hyper-Permeabilitäts-Ödem, welches entweder primär durch direkte Einwirkung einer Noxe oder sekundär als Folgeerscheinung anderer Erkrankungen entstehen kann. Die häufigsten Lungenschädigungen bei ALI, ARDS und beim Hyper-Permeabilitäts- Ödem sind bakterielle und virale Pneumonie, Lungenkontusion, Aspiration von Magensaft, Inhalationstrauma, Rauchgas-Vergiftungen, Beinahe-Ertrinken, Bluttransfusionen, Sepsis, Polytrauma, Massivtransfusion, Kardiopulmonaler Bypass oder großflächige Verbrennungen. Bei diesen Lungenerkrankungen steht die Entzündungsreaktion mit einhergehender Schädigung der Alveolarwände im Vordergrund. Dieser Zustand führt zu einer komplexen Aktivierung von pro- und antiinflammatorischen Immunvorgängen die zur entzündlichen Schädigung des Alveolarepithels und des Gefäßendothels führen. Die Folge sind der Verlust an Alveolozyten und Surfactant, das Auftreten eines capillary leaks mit Austritt von Plasmaproteinen und interstitieller Ödembildung. Die entzündlichen Veränderungen sind typischerweise fleckförmig und inhomogen über die gesamte Lunge verteilt. Infiltration, interstitielles und alveoläres Ödem führen letztendlich zu Atelektasen und den klinischen Zeichen der arteriellen Hypoxämie und der pulmonalen Hypertension. Derartige Entzündungsreaktionen haben bei der Höhenerkrankung keine pathologische Bedeutung.

Die Inzidenz einer klinisch manifesten pulmonalen Form der Höhenkrankheit liegt oberhalb von 3500 m bei rund 15%, wobei die Letalität bei 44 % der unbehandelten Patienten liegt.

Die Inzidenz der Höhenkrankheit korreliert nicht mit der VO₂ₘₐₓ, dem Trainingszustand, dem Blutdruck, der Ernährung, dem Zigarettenrauchen oder dem Lebensalter (im Gegensatz zum akuten Lungenschaden (ALI/ARDS), bei dem v.a. Zigarettenrauchen und ein hohes Lebensalter deutliche Risikofaktoren darstellen), wohl aber zum Teil mit der individuellen hypoxischen Atemantwort (HVR) und mit dem Bergziel bzw. der Steiggeschwindigkeit.

Vorzugsweise betrifft die vorliegende Erfindung ein Peptid, welches aus 7-17 benachbarten Aminosäuren besteht und das Hexamer TPEGAE (SEQ ID Nr. 2) umfasst, wobei das Peptid keine TNF-Rezeptor-Bindungsaktivität aufweist und zyklisiert ist, zur Behandlung der pulmonalen Form der Höhenkrankheit.

Eine besonders bevorzugte Ausführungsform der vorliegenden Erfindung betrifft ein zyklisiertes Peptid, bestehend aus einer Sequenz aufeinanderfolgender Aminosäuren, ausgewählt aus der Gruppe bestehend aus
- QRETPEGAEAKPWY (SEQ ID Nr. 3)
- PKDTPEGAELKPWY (SEQ ID Nr. 4)
- CGQRETPEGAEAKPWYC (SEQ ID Nr. 1) und
- CGPKDTPEGAELKPWYC (SEQ ID Nr. 5)
und Fragmenten von mindestens 7 Aminosäuren davon, welche Fragmente das Hexamer TPEGAE aufweisen, zur Anwendung bzw. zur Herstellung eines Medikaments zur Behandlung der pulmonalen Form der Höhenkrankheit.

Vorzugsweise umfasst das Peptid die Aminosäuresequenz CGQRETPEGAEAKPWYC (SEQ ID Nr. 1) und ist über die C-Reste zyklisiert. Dieses besonders bevorzugte Peptid hat somit folgende Aminosäuresequenz (SEQ ID Nr. 1) (NH₂)Cys-Gly-Gln-Arg-Glu-Thr-Pro-Glu-Gly-Ala-Glu-Ala-Lys-Pro-Trp-Tyr-Cys(COOH).

Die Zyklisierung der erfindungsgemäßen Peptide kann dabei z.B. entweder über eine direkte Zyklisierung über eine Disulfidbrücke zwischen den beiden C-Resten am N- und C-Terminus erreicht werden oder aber indem das Peptid über beide Cysteine an eine Trägersubstanz gekoppelt wird. Dabei werden in den erfindungsgemäßen Peptiden die Cysteinreste vorzugsweise am Anfang und am Ende des Moleküls vorgesehen. Auch andere funktionelle Gruppen, die eine Zyklisierung des Peptids erreichen, können eingesetzt werden, z.B. indem eine Säuregruppe mit einem Amin oder Alkohol zu einem Amid- oder Ester-Ringschluss führt (hierbei können z.B. die Aminosäuren Asparaginsäure und Glutaminsäure mit Serin, Threonin, Tyrosin, Asparagin, Glutamin oder Lysin, vorzugsweise intramolekular zyklisiert werden). Die Zyklisierung des Peptids erfolgt vorzugsweise durch eine Disulfidbrücke zwischen den C-Resten des Peptids (wenn vorhanden).

Weitere bevorzugte erfindungsgemäße Peptide sind daher beispielsweise CGQKETPEGAEAKPWYC (SEQ ID Nr. 6), CGQRETPEGAEARPWYC (SEQ ID Nr. 7), CGQRETPEGAEAKPC (SEQ ID Nr. 8), CQRETPEGAEAKPWYC (SEQ ID Nr. 9) oder CGQRETPEGAEAKFWYC (SEQ ID Nr. 10).

Die Zyklisierung kann aber auch durch Bindung des Peptids an Trägersubstanzen erfolgen. Als derartige Zyklisierungs-Trägersubstanzen kommen alle gängigen pharmazeutisch verwendbaren Substanzen in Frage, die in der Lage sind, z.B. mit den SH-Gruppen der Cysteine (oder mit anderen natürlicherweise vorhandenen oder artifiziell eingeführten chemisch reaktiven Gruppen des Peptids) eine kovalente Bindung einzugehen, wobei gängige Trägerproteine, wie Keyhole limpet hemocyanin (KLH), Tetanus-Toxin etc., sich besonders eignen. Auch können am Träger benachbarte bifunktionelle Reste vorgesehen werden (z.B. Säuregruppe neben Amin- oder Alkoholgruppe). In diesem Zusammenhang ist wichtig, dass mit "Zyklisierung" sowohl der intramolekulare Ringschluss als auch die Einbindung eines Trägers umfasst ist (von dem das gebundene Peptid hervorragt (indem der N- und der C-Terminus des Peptids an den Träger gebunden ist)), wobei das derart zyklisierte Peptid die zyklische Raumstruktur zeigt und entsprechend stabilisiert ist.

Bevorzugter Weise wird das erfindungsgemäße Peptid zur Behandlung der pulmonalen Form der Höhenkrankheit in einer pharmazeutischen Zusammensetzung zur Verfügung gestellt, die einen pharmazeutisch akzeptablen Träger umfasst. Dabei ist die pharmazeutische Zusammensetzung vorzugsweise in einer Form hergerichtet, die zur Verabreichung am Menschen geeignet ist.

Der Ausdruck "eine pharmazeutische Zusammensetzung" bezieht sich auf jede Zusammensetzung, die ein Peptid, wie oben definiert, umfasst (natürlich auch geeignete (i.e. nicht miteinander negativ interferierende) Gemische des erfindungsgemäßen Peptids mit weiteren Wirksubstanzen; es ist jedoch bevorzugt, das erfindungsgemäße Peptid als einzige Wirksubstanz vorzusehen, welche die hierin beschriebenen Zustände verhindert, verbessert oder heilt. Insbesondere bezieht sich der Ausdruck "eine pharmazeutische Zusammensetzung" auf eine Zusammensetzung, die ein Peptid, wie oben beschrieben, und einen pharmazeutisch akzeptablen Träger oder Exzipienten (beide Ausdrücke können austauschbar verwendet werden) aufweist. Geeignete Beispiele von Trägern oder Exzipienten, die dem Fachmann bekannt sind, sind Wasser, Kochsalzlösung, Natriumphosphat, Natriumacetat, Natriumcarbonat, Zitrat, Glycin, Glycylglycin, Histidin, Lysin, Arginin, TRIS und Natriumcitrat oder Mischungen davon. Natürlich können auch Ringer-Lösung, Dextrose-Lösung oder Lösungen nicht reduzierbarer Zucker eingesetzt werden; demgemäß eignen sich auch Mannit, Trehalose, Saccharose, Sorbit, Fruchtzucker, Maltose, Lactose oder Dextran, Hank-Lösung, fixierte Öle, Ethyloleat, 5% Dextrose in Kochsalzlösung, Substanzen, die die Isotonie und die chemische Stabilität verbessern, Puffer und Konservierungsmittel als derartige Träger. Andere geeignete Träger inkludieren jeden Träger, der nicht selbst die Produktion von Antikörpern, die für das die Zusammensetzung erhaltende Individuum schädlich sind, induziert, wie Proteine, Polysaccharide, Polymilchsäuren, Polyglykolsäuren, polymere Aminosäuren und Aminosäure-Copolymere. Bei der Formulierung der erfindungsgemäßen pharmazeutischen Zusammensetzung sind natürlich die einschlägigen Richtlinien (z.B. die (europäische oder US) Pharmakopöe) zu beachten. Dabei kann das in der erfindungsgemäßen Zusammensetzung vorgesehene Peptid auch durch direkte kovalente Bindung an diese Träger zyklisiert werden.

Die erfindungsgemäße pharmazeutische Zusammensetzung kann (als Medikament) mit jedem im Wissen des Fachmanns liegenden geeigneten Verfahren verabreicht werden, insbesondere ist bevorzugt, das erfindungsgemäß zu verwendende Peptid bzw. die erfindungsgemäße Zusammensetzung in die Lunge zu verabreichen. Der bevorzugte Verabreichungsweg ist Inhalation (durch Aerosole) aber auch die intravenöse Gabe, Instillation, orale Gabe oder Kombinationen daraus. Bei inhalativer, parenteraler oder oraler Verabreichung wird das Medikament dieser Erfindung in Dosiseinheitsform, wie als Lösung, Suspension oder Emulsion, in Verbindung mit den oben definierten pharmazeutisch akzeptablen Exzipienten formuliert. Die Dosierung und Verabreichungsart kann jedoch natürlich auch im Einzelfall vom jeweiligen Individuum abhängen.

Dabei wird die jeweils erforderliche wirksame Menge an das Individuum, das die Verabreichung benötigt, verabreicht. Dabei ist die "wirksame Menge" als eine Menge zu verstehen, die wirksam genug ist, um den beabsichtigten therapeutischen oder prophylaktischen Effekt zu erzielen, also z.B. eine weitere Verschlechterung der Erkrankung zu verhindern oder wirksam zu behandeln. Dabei wird meist von einem durchschnittlichen Patienten ausgegangen, jedoch können die tatsächlichen wirkungsvollen Mengen der Komponenten in der Zusammensetzung so formuliert werden, dass die Art der Verabreichung und das Alter, Gewicht, Zustand des Patienten und Grad und Fortschritt der Erkrankung berücksichtigt werden (z.B. mittels eines passenden, herkömmlichen pharmakologischen Protokolls).

Vorzugsweise ist daher der pharmazeutisch akzeptable Träger in der erfindungsgemäßen Zusammensetzung ausgewählt aus Wasser (besonders bevorzugt: Wasser für Injektion), Kochsalz, Natriumphosphat, Natriumacetat, Natriumcarbonat, Zitrat, Glycin, Glycylglycin, Histidin, Lysin, Arginin, TRIS, Natriumcitrat, Ringer-Lösung, Dextrose, Mannit, Trehalose, Saccharose, Sorbit, Fruchtzucker, Maltose, Lactose oder Dextran, Hank-Lösung, fixierte Öle, Ethyloleat, Substanzen, die die Isotonie und die chemische Stabilität verbessern, Konservierungsmittel, pharmazeutisch akzeptable Proteine, Polysaccharide, Polymilchsäuren, Polyglykolsäuren, polymere Aminosäuren und Aminosäure-Copolymere.

Das erfindungsgemäße Medikament kann beispielsweise so verabreicht werden, dass das Peptid der vorliegenden Erfindung in einer Dosis von zwischen 1 µg/kg und 10 mg/kg, mehr bevorzugt zwischen 10 µg/kg und 5 mg/kg, am meisten bevorzugt zwischen 0,1 und 2 mg/kg, gegeben wird. Vorzugsweise wird es als Bolus-Dosis gegeben. Es kann aber auch eine kontinuierliche Inhalation oder Infusion oder eine Verabreichung mittels wiederholter Gabe verwendet werden.

Besonders bevorzugte erfindungsgemäße Zusammensetzungen enthalten das Peptid in einer Menge von 1 µg bis 10 g, vorzugsweise von 10 µg bis 1 g, insbesondere von 1 mg bis 100 mg.

Besonders bevorzugte erfindungsgemäße Zusammensetzungen in flüssiger Form enthalten das Peptid in einer Menge von 1 µg bis 10 g, vorzugsweise von 10 µg bis 1 g, insbesondere von 1 mg bis 100 mg, und liegen in einem Volumen von 0,5 bis 10 ml, insbesondere in einem Volumen von 1 bis 5 ml, vor.

Die erfindungsgemäße Zusammensetzung kann vorzugsweise auch in Trockenform mittels Pulverinhalator verabreicht werden. Beispiele für derartige Pulverinhalatoren, die für die vorliegende Erfindung eingesetzt werden können, sind in den US-Patenten 4.995.385 und 4.069.819 beschrieben; bereits etablierte Produkte sind SPINHALER^{®}, ROTAHALER^{®}, FLOWCAPS^{®}, INHALATOR^{®}, DISKHALER^{®} und AEROLIZER^{®}.

Die erfindungsgemäße Zusammensetzung kann vorzugsweise auch als Aerosol mittels Flüssigkeitsvernebler verabreicht werden. Beispiele für derartige Flüssigkeitsvernebler sind etablierte Produkte wie Aeroneb^{®} und Pari^{®}.

Gemäß einer bevorzugten Ausführungsform ist die erfindungsgemäße Zusammensetzung dadurch gekennzeichnet, dass das Peptid in einer vernebelbaren Pulverformulierung oder in einer vernebelbaren Flüssigformulierung vorliegt.

Die Erfindung wird anhand der nachfolgenden Beispiele und der Zeichnungsfiguren, auf die sie aber selbstverständlich nicht beschränkt ist, näher erläutert.

Es zeigen:

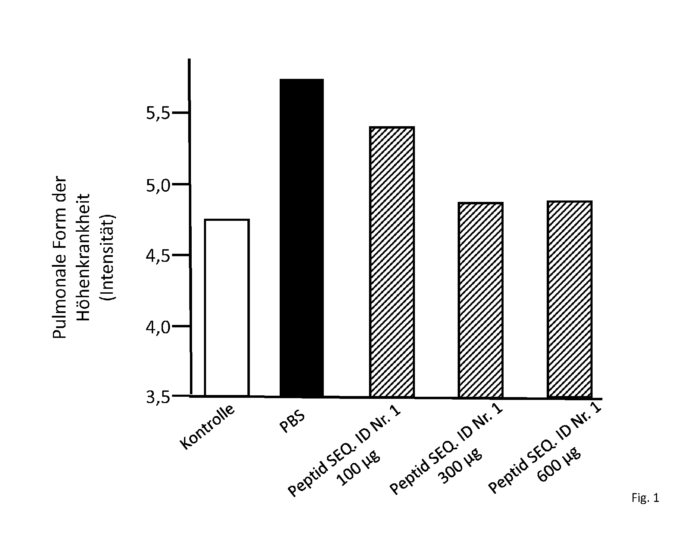

Figur 1: Die Intensität der pulmonalen Form der Höhenkrankheit in Ratten wurde 4 Stunden nach intratrachealer Gabe von Kochsalzlösung bzw. Peptid SEQ ID Nr. 1 bestimmt. Kontrolle: Kontroll-Ratten unter Bedingungen von normalen Sauerstoff- und Luftdruckwerten. PBS: Ratten unter Bedingungen von verringertem Sauerstoff und Luftdruck und intratrachealer Gabe von Kochsalzlösung. Peptid SEQ ID Nr. 1 100 µg: Ratten unter Bedingungen von verringertem Sauerstoff und Luftdruck und intratrachealer Gabe von 100 µg Peptid SEQ ID Nr. 1. Peptid SEQ ID Nr. 1 300 µg: Ratten unter Bedingungen von verringertem Sauerstoff und Luftdruck und intratrachealer Gabe von 300 µg Peptid Seq. ID No. 1. Peptid SEQ ID Nr. 1 600 µg: Ratten unter Bedingungen von verringertem Sauerstoff und Luftdruck und intratrachealer Gabe von 600 µg Peptid SEQ ID Nr. 1.

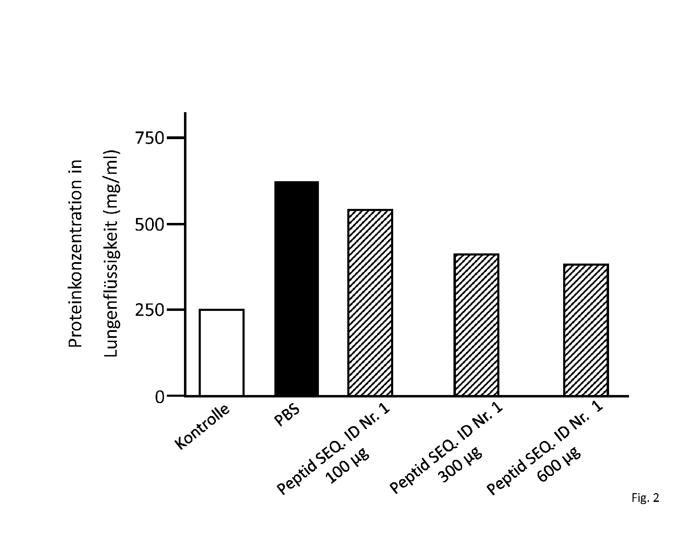

Figur 2: Der Gehalt an Protein in der Lungenflüssigkeit in Ratten wurde 4 Stunden nach intratrachealer Gabe von Kochsalzlösung bzw. Peptid SEQ ID Nr. 1 bestimmt. Kontrolle: Kontroll-Ratten unter Bedingungen von normalen Sauerstoff- und Luftdruckwerten. PBS: Ratten unter Bedingungen von verringertem Sauerstoff und Luftdruck und intratrachealer Gabe von Kochsalzlösung. Peptid SEQ ID Nr. 1 100 µg: Ratten unter Bedingungen von verringertem Sauerstoff und Luftdruck und intratrachealer Gabe von 100 µg Peptid SEQ ID Nr. 1. Peptid SEQ ID Nr. 1 300 µg: Ratten unter Bedingungen von verringertem Sauerstoff und Luftdruck und intratrachealer Gabe von 300 µg Peptid SEQ ID Nr. 1. Peptid SEQ ID Nr. 1 600 µg: Ratten unter Bedingungen von verringertem Sauerstoff und Luftdruck und intratrachealer Gabe von 600 µg Peptid SEQ ID Nr. 1.

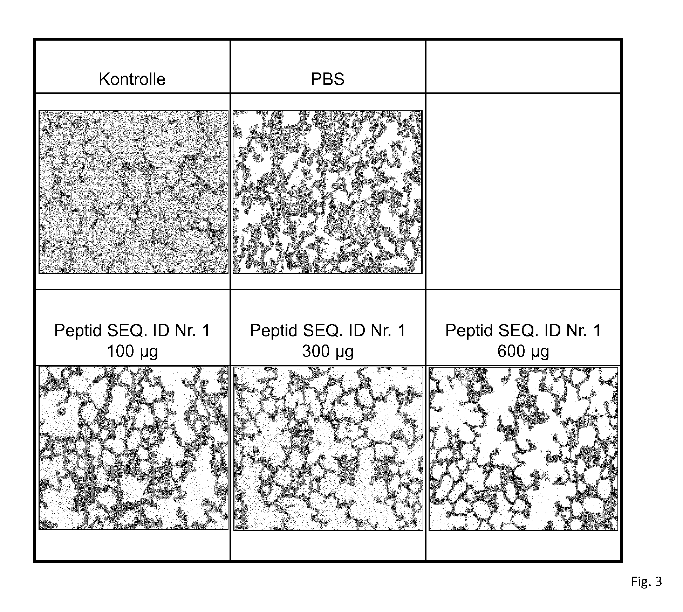

Figur 3: Histologisches Erscheinungsbild von Lungengewebe in Ratten 4 Stunden nach intratrachealer Gabe von Kochsalzlösung bzw. Peptid SEQ ID Nr. 1. Kontrolle: Kontroll-Ratten unter Bedingungen von normalen Sauerstoff- und Luftdruckwerten. PBS: Ratten unter Bedingungen von verringertem Sauerstoff und Luftdruck und intratrachealer Gabe von Kochsalzlösung. Peptid SEQ ID Nr. 1 100 µg: Ratten unter Bedingungen von verringertem Sauerstoff und Luftdruck und intratrachealer Gabe von 100 µg Peptid Seq. ID No. 1. Peptid SEQ ID Nr. 1 300 µg: Ratten unter Bedingungen von verringertem Sauerstoff und Luftdruck und intratrachealer Gabe von 300 µg Peptid SEQ ID Nr. 1. Peptid SEQ ID Nr. 1 600 µg: Ratten unter Bedingungen von verringertem Sauerstoff und Luftdruck und intratrachealer Gabe von 600 µg Peptid SEQ ID Nr. 1.

### Beispiel 1:

Verwendung von Peptid SEQ ID Nr. 1 zur Behandlung der pulmonalen Form der Höhenkrankheit

Mit dem vorliegenden Beispiel wird in einem experimentellen Rattenmodell der Höhenkrankheit gezeigt, dass die erfindungsgemäße Zielstellung erreicht wurde, indem an der pulmonalen Form der Höhenkrankheit leidenden Ratten das synthetische Peptid (SEQ ID Nr. 1) verabreicht wurde. Körperliche Anstrengung unter Bedingungen von verringertem Sauerstoff und Luftdruck, wie sie in großen Höhen vorkommen, sind die 2 Hauptfaktoren, die zur Entwicklung der pulmonalen Form der Höhenkrankheit führen. Daher simuliert das gewählte Rattenmodell, in welchem die Ratten unter Bedingungen von verringertem Sauerstoff und verringertem Luftdruck körperliche Tätigkeit verrichteten, einen körperlich anstrengenden Aufstieg auf große Höhen. Dies geschieht ohne eine vorhergehende Akklimatisation vorzunehmen. Dies entspricht dem Szenario, wie es bei Bergsteigern anzutreffen ist, die in großen Höhen an der pulmonalen Form der Höhenkrankheit leiden. Im verwendeten Model entwickeln die Ratten die für die pulmonale Form der Höhenkrankheit typischen Symptome, wie an der "Intensität der pulmonalen Form der Höhenkrankheit", der erhöhten Proteinkonzentration in der Lungenflüssigkeit und dem histologischen Erscheinungsbild des Lungengewebes ablesbar ist. Es ist weiter anzumerken, dass der Lungenschaden in diesem Modell nicht durch Verabreichung von Endotoxinen, Mikroben oder anderen die Lunge schädigenden Agenzien verursacht wird. Eine verstärkte Inflammation der Lunge tritt nicht auf. Es wurde auch kein spezieller Rattenstamm für dieses Experiment verwendet. Daher ist dieses Rattenmodell gut geeignet ein Medikament zur Behandlung der pulmonalen Form der Höhenkrankheit zu untersuchen.

### Methode

Laborratten (Sprague Dawley Ratten) verrichteten durch externe Stimulation 48 Stunden lang körperliche Tätigkeit unter Bedingungen von verringertem Sauerstoff und Luftdruck. Hier wurde der Luftdruck auf einen Wert unter 430 Torr reduziert, sodass eine Höhe von über 4500 m simuliert wurde. Eine vorhergehende Akklimatisation der Ratten an die Höhe von über 4500 m wurde nicht vorgenommen. Während dieser Zeit konnten die Ratten alle 4 Stunden eine 15-20 minütige Pause einlegen um Wasser und Nahrung aufzunehmen. Nach 48 h körperlicher Tätigkeit auf der simulierten Höhe von über 4500 m wurden die Ratten intratracheal mit 300 µl/Versuchstier Peptid SEQ ID Nr. 1 (100 µg, 300 µg und 600 µg) oder 300 µl Kochsalzlösung behandelt. Anschließend verbrachten die Ratten weitere 4 Stunden unter Bedingungen von verringertem Sauerstoff und Luftdruck. Dann wurden die Lungen entnommen und die Intensität der pulmonalen Form der Höhenkrankheit ermittelt (Figur 1), der Gehalt an Protein in der Lungenflüssigkeit ermittelt (Figur 2) und das histologische Erscheinungsbild des Lungengewebes bestimmt (Figur 3).

### Ergebnis

Die Untersuchung ergab, dass die intratracheale Gabe von Peptid SEQ ID Nr. 1 an Laborratten, welche den Bedingungen von verringertem Luftdruck und verringerter Sauerstoffkonzentration ausgesetzt waren, zu Verringerung der Intensität der pulmonalen Form der Höhenkrankheit führte (Figur 1). Dies konnte für 100 µg/Laborratte und 600 µg/Laborratte und vorzugsweise für 300 µg/Laborratte Peptid SEQ ID Nr. 1 nachgewiesen werden.

Die Untersuchung ergab weiters, dass die intratracheale Gabe von Peptid SEQ ID Nr. 1 an Laborratten, welche den Bedingungen von verringertem Luftdruck und verringerter Sauerstoffkonzentration ausgesetzt waren, zu Verringerung der Proteinkonzentration in der Lungenflüssigkeit führte (Figur 2). Dies konnte für 100 µg/Laborratte und 600 µg/Laborratte und vorzugsweise für 300 µg/Laborratte Peptid SEQ ID Nr. 1 nachgewiesen werden.

Die histologische Untersuchung ergab, dass die mit Kochsalzlösung behandelten Ratten geschwollenes Lungengewebe mit roten Blutkörperchen aufwiesen, wobei das Lungengewebe in Ratten nach Gabe von Peptid SEQ ID Nr. 1 vergleichbar mit gesundem Lungengewebe der Kontroll-Ratten war.

### Beispiel 2:

### Ex vivo Beurteilung der pro-inflammatorischen Eigenschaften des Peptids SEQ ID Nr. 1 im menschlichen Vollblut.

Eine pharmakologische ex-vivo-Sicherheitsstudie hinsichtlich des Peptids SEQ ID Nr. 1 im menschlichen Vollblut wurde durchgeführt, um festzustellen, ob das Peptid SEQ ID Nr. 1 zur Freisetzung des pro-inflammatorischen Markers Interleukin-6 (IL-6) aus frischem Vollblut führt (d.h. ob Peptid SEQ ID Nr. 1 TNF-spezifische entzündliche Aktivität (i.e. TNF-Rezeptor-Bindungsaktivität) zeigt oder nicht). In dieser Studie ist frisches Vollblut verwendet worden, hierbei handelt es sich um ein anerkanntes Vorhersagemodell für die Beurteilung der Entzündungsreaktion in vivo.

### Zusammenfassung der Methodik

Es war das Ziel dieser Studie, die proinflammatorische Signal-Kapazität des Peptids SEQ ID Nr. 1 zu bestimmen. Dabei wurden Vollblut-Kulturen verwendet und die Sekretion von Interleukin-6 (IL-6), ein sehr sensitiver Marker für proinflammatorische Stimulation, mittels ELISA quantifiziert.

Testsystem 25 ml frisch entnommenes heparinisiertes Blut von 5 gesunden Probanden (GP) wurde in den Tests verwendet.

Prüfgegenstand
- Identifikation:: Peptid SEQ ID Nr. 1 (Dosis: 1 ng/ml bis 10 µg/ml; einmalige Verabreichung in Lösung)
- Beschreibung:: Weißes Pulver, Reinheit 96%

### Vollblut-Kulturen

Vollblut (VB)-Kulturen wurden durch Pipettieren von 1 ml VB in Vertiefungen von 24-Näpfchen-Platten durchgeführt. In jedem Experiment wurden unstimulierte und stimulierte Kontroll-Kulturen inkludiert.

Wenn möglich wurden die zu untersuchenden Substanzen und Stimulanzien immer in gleichem Volumen in jedem Näpfchen in einem gegebenen Experiment verwendet, welches nicht größer als 10% des Gesamtvolumens in einem Näpfchen ist. Unstimulierte Kontrollen erfolgten mit PBS. Volumeneinstellung und Verdünnungen für verschiedene Behandlungen wurden ebenfalls mit PBS durchgeführt.

Der Inhalt jedes Näpfchens wurde gemischt und die Platten bei 37°C und 5% CO₂ für 24 Stunden inkubiert. Nach der Inkubation wurde der Inhalt jedes Näpfchens in ein frisches 1,5 ml Mikroröhrchen überführt und bei 8000 bis 9000 x g für 15 Minuten zentrifugiert. Der Überstand jeder Probe wurde einzeln auf zwei 1,5 ml Reaktionsgefäße aufgeteilt und bei - 20°C bis zum Gebrauch gelagert.

### Nachweis von Interleukin-6

Interleukin-6 wurde mittels eines spezifischen ELISA quantifiziert (Human IL-6 ELISA-Set, BD Biosciences, Cat. Nr. 555220) unter Einsatz eines Anti-Human-IL-6 Antikörpers als Fänger-Antikörper, eines biotinylierten anti-human IL-6 Detektionsantikörpers, Avidin-Meerrettichperoxidase-Konjugat als Enzym-Reagenz und rekombinantem IL-6 als Standard. Absorptionsmessung bei 450 nm wurde mit dem Packard FusionReader durchgeführt.

### Datenanalyse

Die Ergebnisse jeder Platte wurden gespeichert und mit der FusionDataAnalysis Software ausgewertet.

### Zusammenfassung der Ergebnisse der Studie

Es war das Ziel dieser Studie, die pro-inflammatorische Signalisierungs-Kapazität des Peptids SEQ ID Nr. 1 zu bestimmen. Vollblut-Kulturen wurden verwendet und die Sekretion von IL-6, einem sehr sensitiven Marker für entzündliche Pro-Stimulation, wurde mittels ELISA quantifiziert.

Vollblutproben von fünf gesunden Probanden wurden entweder unstimuliert belassen (negative Kontrolle), stimuliert mit hohen und niedrigen Dosen von LPS (positive Kontrollen) oder mit dem Peptid in neun halblogarithmischen Verdünnungen von 10 µg/ml bis 1 ng/ml inkubiert. Die Ergebnisse sind in der nachfolgenden Tabelle dargestellt:

**Tabelle: Freisetzung von Interleukin-6 aus frischem Vollblut bei Zugabe von Peptid SEQ ID Nr. 1 und LPS**

| | Peptid SEQ ID Nr. 1 Positive Kontrolle (LPS) | |
|---|---|---|
| Konzentration | Konzentration von IL-6 (pg/ml, n = 5) | |
| 0 (Negativ-kontrolle) | weniger als 0,5 | weniger als 0,5 |
| 10 mg/ml | weniger als 0,5 | 195,640 |
| 1 mg/ml | weniger als 0,5 | 108,370 |
| 3 ng/ml | weniger als 0,5 | 34,867 |
| 1 ng/ml | weniger als 0,5 | nicht bestimmt |

Die Ergebnisse zeigen deutlich, dass das Peptid SEQ ID Nr. 1 keine nachweisbare Menge von IL-6-Sekretion bei keinem der getesteten Konzentrationen induzierte. Die positiven Kontrollen (LPS) führten zu einer starken Induktion der IL-6-Sekretion.

### Diskussion

Die Versuche wurden durchgeführt, um festzustellen, ob das Peptid SEQ ID Nr. 1 die Induktion einer pro-inflammatorischen Kaskade vermittelt. Readout-Parameter war die induzierte Sekretion von IL-6 in Vollblut-Kulturen aus fünf gesunden Spendern. Die Ergebnisse zeigten deutlich, dass das Peptid SEQ ID Nr. 1 kein nachweisbares Niveau von IL-6 in den Spenderkulturen induzierte. Damit ist bewiesen, dass das Peptid SEQ ID Nr. 1 keine pro-inflammatorische Antwort in dem gewählten ex vivo-Modell induziert und somit keine TNF-Rezeptor-Bindungsaktivität aufweist. Dieser Test kann für beliebige Varianten des erfindungsgemäßen Peptids angewendet werden, um das Merkmal der Freiheit von TNF-Rezeptor-Bindungsaktivität festzustellen.

### Zusammenfassung der Sequenzen:

- SEQ ID Nr. 1: CGQRETPEGAEAKPWYC
- SEQ ID Nr. 2: TPEGAE
- SEQ ID Nr. 3: QRETPEGAEAKPWY
- SEQ ID Nr. 4: PKDTPEGAELKPWY
- SEQ ID Nr. 5: CGPKDTPEGAELKPWYC
- SEQ ID Nr. 6: CGQKETPEGAEAKPWYC
- SEQ ID Nr. 7: CGQRETPEGAEARPWYC
- SEQ ID Nr. 8: CGQRETPEGAEAKPC
- SEQ ID Nr. 9: CQRETPEGAEAKPWYC
- SEQ ID Nr. 10: CGQRETPEGAEAKFWYC

## Patentansprüche

1. Peptid, welches aus 7-17 benachbarten Aminosäuren besteht und das Hexamer TX₁EX₂X₃E umfasst, wobei X₁, X₂ und X₃ jede natürliche oder nicht natürliche Aminosäure sein kann, wobei das Peptid keine TNF-Rezeptor-Bindungsaktivität aufweist und zyklisiert ist, zur Anwendung zur Behandlung und Vermeidung der pulmonalen Form der Höhenkrankheit.

2. Peptid nach Anspruch 1, wobei das Peptid aus 7-17 benachbarten Aminosäuren besteht und das Hexamer TPEGAE umfasst.

3. Peptid nach Anspruch 1 oder 2, wobei das zyklisierte Peptid aus einer Sequenz aufeinander folgender Aminosäuren, ausgewählt aus der Gruppe bestehend aus QRETPEGAEAKPWY, PKDTPEGAELKPWY, CGQRETPEGAEAKPWYC, CGPKDTPEGAELKPWYC und Fragmenten von mindestens 7 Aminosäuren davon, welche Fragmente das Hexamer TPEGAE aufweisen, besteht.

4. Peptid nach einem der Ansprüche 1 bis 3, **dadurch gekennzeichnet, dass** das Peptid die Aminosäuresequenz CGQRETPEGAEAKPWYC umfasst und über die C-Reste zyklisiert ist.

5. Peptid nach einem der Ansprüche 1 bis 4, **dadurch gekennzeichnet, dass** das Peptid durch eine Disulfidbrücke zwischen den C-Resten zyklisiert ist.

6. Pharmazeutische Zusammensetzung, enthaltend ein Peptid wie in einem der Ansprüche 1 bis 5 definiert, **dadurch gekennzeichnet, dass** es in einer pharmazeutischen Zusammensetzung formuliert ist, die zur Verabreichung am Menschen geeignet ist, und einen pharmazeutisch akzeptablen Träger umfasst.

7. Pharmazeutische Zusammensetzung nach Anspruch 6, **dadurch gekennzeichnet, dass** sie einen pharmazeutisch akzeptablen Träger umfasst, der ausgewählt ist aus Wasser, insbesondere Wasser für Injektion, Kochsalz, Natriumphosphat,
Natriumacetat, Natriumcarbonat, Zitrat, Glycin, Glycylglycin, Histidin, Lysin, Arginin, TRIS, Natriumcitrat, Ringer-Lösung, Dextrose, Mannit, Trehalose, Saccharose, Sorbit, Fruchtzucker, Maltose, Lactose oder Dextran, Hank-Lösung, fixierte Öle, Ethyloleat, Substanzen, die die Isotonie und die chemische Stabilität verbessern, Konservierungsmittel, pharmazeutisch akzeptable Proteine, Polysaccharide, Polymilchsäuren, Polyglykolsäuren, polymere Aminosäuren und Aminosäure-Copolymere.

8. Pharmazeutische Zusammensetzung nach Anspruch 6 oder 7, **dadurch gekennzeichnet, dass** sie das Peptid in einer Menge von 1 µg bis 10 g, vorzugsweise von 10 µg bis 1 g, insbesondere von 1 mg bis 100 mg, umfasst.

9. Pharmazeutische Zusammensetzung nach einem der Ansprüche 6 bis 8, **dadurch gekennzeichnet, dass** sie in flüssiger Form vorliegt und das Peptid in einer Menge von 1 µg bis 10 g, vorzugsweise von 10 µg bis 1 g, insbesondere von 1 mg bis 100 mg, umfasst und in einem Volumen von 0,5 bis 10 ml, insbesondere in einem Volumen von 1 bis 5 ml, vorliegt.

10. Pharmazeutische Zusammensetzung nach einem der Ansprüche 6 bis 9, **dadurch gekennzeichnet, dass** das Peptid in einer vernebelbaren Pulverformulierung oder in einer vernebelbaren Flüssigformulierung vorliegt.
